(19)

Europäisches
Patentamt
European
Patent Office
Office européen
des brevets

(11) **EP 4 354 444 A1**

(12) **EUROPEAN PATENT APPLICATION**

(43) Date of publication:
**17.04.2024 Bulletin 2024/16**

(21) Application number: **23197703.4**

(22) Date of filing: **15.09.2023**

(51) International Patent Classification (IPC):
**G16B 30/00** (2019.01)    **G16B 30/10** (2019.01)

(52) Cooperative Patent Classification (CPC):
**G16B 30/00; G16B 30/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.10.2022 IN 202221058653**

(71) Applicant: **Tata Consultancy Services Limited Maharashtra (IN)**

(72) Inventors:
• **WALIA, VIDUSHI**
**201309 Noida - Uttar Pradesh (IN)**
• **VANGALA GOVINDAKRISHNAN, SAIPRADEEP**
**560 009 Bangalore - Karnataka (IN)**
• **SIVADASAN, NAVEEN**
**500081 Hyderabad - Telangana (IN)**
• **SRINIVASAN, RAJGOPAL**
**500081 Hyderabad - Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR IDENTIFYING CANDIDATE GENOME SEQUECNCES BY ESTIMATING COVERAGE**

(57) This disclosure relates generally to identifying candidate genome sequences. Next generation sequencing (NGS) is a massively parallel sequencing technique for identifying candidate genome sequences. The current state-of-the-art techniques for identifying candidate genome sequences does not efficiently address the problem of distributing abundance values across several related strains that are present in the reference under the same species. The disclosed technique proposes a technique for identifying candidate genome sequences by estimating coverage. The disclosed technique includes a local search-based optimization to compute maximum likelihood-based estimates using constrains on coverage/cardinality thresholds for identifying candidate genome sequences.

FIG. 2

200

EP 4 354 444 A1

**Description**

CROSS-REFERENCE TO RELATED APPLICATIONS AND PRIORITY

The present application claims priority to Indian application, Application No. 202221058653, filed in India on October 13, 2022.

TECHNICAL FIELD

The disclosure herein generally relates to identifying candidate genome sequences and, more particularly, to identifying candidate genome sequences by estimating coverage.

BACKGROUND

Next generation sequencing (NGS) is a massively parallel sequencing technique, wherein sequencing in molecular biology refers to study of genomes and the proteins. The NGS techniques allow high-throughput speed and scalability during sequencing. A NGS read sample can be performed at several levels including a genomic read sample, a transcriptomic read sample, a metagenomic read sample etc. NGS is a powerful platform that has enabled the sequencing of thousands to millions of DNA molecules simultaneously. NGS technologies have greatly facilitated culture independent analysis of microbiome, which has led to dramatic increase in the number and scope of metagenomic studies.
Considering an example of metagenomic sequencing - the metagenomics sequencing of microbial sample during genomic research enables detailed characterization of environmental or host-associated microbial communities in the microbial sample. The detailed characterization of microbial communities can be used for several applications including clinical study, disease study, study of environmental habitats, characterizing an industrial product, and study of host-pathogen interactions.
An essential prerequisite for any metagenomic sequencing is to disentangle the microbial sample at lower ranks of taxonomy such as species/strain with precise measurements of their abundances. However, the metagenomic samples are usually diverse and contain strains that share high genomic similarity, thus making the metagenomic analysis tremendously challenging, especially at the higher resolutions of species and strain.
The state-of-the-art techniques are not very efficient for distributing the relative abundance values across the related strains that are present under the same species. Of the existing techniques - Maximum Likelihood Estimation (MLE) approach is very popular and efficient for species level abundance estimation. However, MLE does not efficiently address the problem of distributing abundance values across several related strains that are present in the reference under the same species. Hence, most of the current state-of-the-art techniques estimate species level abundances with reasonable accuracy, however the estimation of strain level abundance remains a challenge as the inter-strain similarity is much higher than inter-species similarity.

SUMMARY

Embodiments of the present disclosure present technological improvements as solutions to one or more of the above-mentioned technical problems recognized by the inventors in conventional systems. For example, in one embodiment, a method for identifying candidate genome sequences by estimating coverage is provided.
The system includes a memory storing instructions, one or more communication interfaces, and one or more hardware processors coupled to the memory via the one or more communication interfaces, wherein the one or more hardware processors are configured by the instructions to share, via one or more hardware processors, for receiving a plurality of inputs, via one or more hardware processors, wherein the plurality of inputs comprises: a plurality of reference genome sequences, a plurality of genomic read sequences, and an initial pruning threshold, a termination threshold and an iterative refinement threshold, wherein the termination threshold comprises a coverage threshold and a cardinality threshold. The system is further configured for indexing the plurality of reference genome sequences, via the one or more hardware processors, based on an indexing technique to obtain a reference genome index. The system is further configured for mapping each of genomic read sequence from the plurality of genomic read sequences to the reference genome index, via the one or more hardware processors, based on a read mapping technique to obtain a read mapping, wherein the read mapping denotes a set of matching genome sequences from the plurality of reference genomic sequences mapping to the reference genome index. The system is further configured computing a first relative abundance for the plurality of reference genome sequences, via the one or more hardware processors, based on an abundance computation technique using the read mapping. The system is further configured for computing a first coverage for each of the reference genomic sequences, via the one or more hardware processors, using a coverage computing technique using the first relative abundance and the read mapping. The method further includes selecting a sub-set of candidate genome sequences from the set of candidate genome sequences to obtain a refined read mapping outcome, via the one or more hardware processors, based on a comparison of the first coverage and the initial pruning threshold. The system is further configured for computing a second relative abundance for the sub-set of candidate genome sequences, via the one or more hardware processors, based on the abundance computation technique using the refined read mapping outcome. The system is further configured for computing a final relative abundance, via the one or more hardware processors, based on a refinement technique using the termination threshold and an iterative refinement threshold, wherein the refinement technique comprises: computing a second coverage for each of the sub-set of candidate genome sequences, based on the coverage computing technique using the second relative abundance and performing based on a comparison of the second coverage with the termination threshold and the iterative refinement threshold one of: if the termination threshold condition is satisfied, then estimating the final relative abundance for the sub-set of candidate genome sequences based on the abundance computation technique and the refined read mapping outcome and if the termination threshold condition is not satisfied, then estimating the final relative abundance for the sub-set of candidate genome sequences based on an iterative refinement technique.

In another aspect, a method for identifying candidate genome sequences by estimating coverage is provided. The method includes receiving a plurality of inputs, wherein the plurality of inputs comprises: a plurality of reference genome sequences, a plurality of genomic read sequences, and an initial pruning threshold, a termination threshold and an iterative refinement threshold, wherein the termination threshold comprises a coverage threshold and a cardinality threshold. The method further includes indexing the plurality of reference genome sequences, based on an indexing technique to obtain a reference genome index. The method further includes mapping each of genomic read sequence from the plurality of genomic read sequences to the reference genome index, based on a read mapping technique to obtain a read mapping, wherein the read mapping denotes a set of matching genome sequences from the plurality of reference genomic sequences mapping to the reference genome index. The method further includes computing a first relative abundance for the plurality of reference genome sequences, based on an abundance computation technique using the read mapping. The method further includes computing a first coverage for each of the reference genomic sequences, using a coverage computing technique using the first relative abundance and the read mapping. The method further includes selecting a sub-set of candidate genome sequences from the set of candidate genome sequences to obtain a refined read mapping outcome, based on a comparison of the first coverage and the initial pruning threshold. The method further includes computing a second relative abundance for the sub-set of candidate genome sequences, based on the abundance computation technique using the refined read mapping outcome. The method further includes computing a final relative abundance, based on a refinement technique using the termination threshold and an iterative refinement threshold, wherein the refinement technique comprises: computing a second coverage for each of the sub-set of candidate genome sequences, based on the coverage computing technique using the second relative abundance and performing based on a comparison of the second coverage with the termination threshold and the iterative refinement threshold one of: if the termination threshold condition is satisfied, then estimating the final relative abundance for the sub-set of candidate genome sequences based on the abundance computation technique and the refined read mapping outcome and if the termination threshold condition is not satisfied, then estimating the final relative abundance for the sub-set of candidate genome sequences based on an iterative refinement technique.

In yet another aspect, a non-transitory computer readable medium for identifying candidate genome sequences by estimating coverage is provided. The method includes receiving a plurality of inputs, wherein the plurality of inputs comprises: a plurality of reference genome sequences, a plurality of genomic read sequences, and an initial pruning threshold, a termination threshold and an iterative refinement threshold, wherein the termination threshold comprises a coverage threshold and a cardinality threshold. The method further includes indexing the plurality of reference genome sequences, based on an indexing technique to obtain a reference genome index. The method further includes mapping each of genomic read sequence from the plurality of genomic read sequences to the reference genome index, based on a read mapping technique to obtain a read mapping, wherein the read mapping denotes a set of matching genome sequences from the plurality of reference genomic sequences mapping to the reference genome index. The method further includes computing a first relative abundance for the plurality of reference genome sequences, based on an abundance computation technique using the read mapping. The method further includes computing a first coverage for each of the reference genomic sequences, using a coverage computing technique using the first relative abundance and the read mapping. The method further includes selecting a sub-set of candidate genome sequences from the set of candidate genome sequences to obtain a refined read mapping outcome, based on a comparison of the first coverage and the initial pruning threshold. The method further includes computing a second relative abundance for the sub-set of candidate genome sequences, based on the abundance computation technique using the refined read mapping outcome. The method further includes computing a final relative abundance, based on a refinement technique using the termination threshold and an iterative refinement threshold, wherein the refinement technique comprises: computing a second coverage for each of the sub-set of candidate genome sequences, based on the coverage computing technique using the second relative abundance and performing based on a comparison of the second coverage with the termination threshold and the iterative refinement threshold one of: if the termination threshold condition is satisfied, then estimating the final relative abundance for the sub-set of candidate genome sequences based on the abundance computation technique and the refined read mapping outcome and if the termination threshold condition is not satisfied, then estimating the final relative abundance for the sub-set of candidate genome sequences based on an iterative refinement technique.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for identifying candidate genome sequences by estimating coverage according to some embodiments of the present disclosure.

FIG. 2 is a functional block diagram of the system of FIG. 1, for identifying candidate genome sequences by estimating coverage according to some embodiments of the present disclosure.

FIGS. 3A, FIG.3B and FIG.3C is a flow diagram illustrating a method (300) for identifying candidate genome sequences by estimating coverage in accordance with some embodiments of the present disclosure.

FIG.4 is a flow diagram illustrating a method (400) for performing the iterative refinement technique during for identifying candidate genome sequences by estimating coverage in accordance with some embodiments of the present disclosure.

FIG.5 is a comparison of strain level profiling with different termination thresholds and ordinary MLE (Maximum Likelihood Estimation) on LC dataset using the distance measures.

FIG.6 is a comparison of strain level profiling with different termination thresholds and ordinary MLE on LC dataset.

FIG.7 is a comparison of strain level profiling with different termination thresholds and ordinary MLE on HC1 dataset using the distance measures.

FIG.8 is a comparison of strain level profiling with different termination thresholds and ordinary MLE on HC-1 dataset.

FIG.9 is a comparison of strain level profiling with different termination thresholds and ordinary MLE on HC-2 dataset using the distance measures.

FIG. 10 is a comparison of strain level profiling with different termination thresholds and ordinary MLE on HC-2 dataset.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

An essential prerequisite for any metagenomic sequencing is to disentangle the microbial sample at lower ranks of taxonomy such as species/strain with precise measurements of their abundances. However, the metagenomic samples are usually diverse and contain strains that share high genomic similarity, thus making the metagenomic analysis tremendously challenging, especially at the higher resolutions of species and strain. The current state-of-the-art techniques for identifying candidate genome sequences does not efficiently address the problem of distributing abundance values across several related strains that are present in the reference under the same species. The disclosed technique proposes a technique for identifying candidate genome sequences by estimating coverage. The disclosed technique includes a local search-based optimization to compute maximum likelihood-based estimates using constrains on coverage/cardinality thresholds for identifying candidate genome sequences.

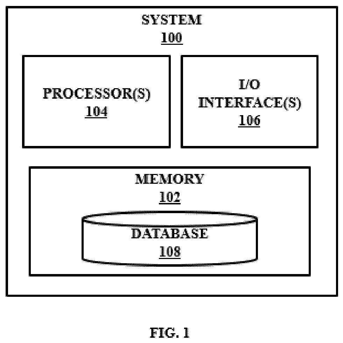

Referring now to the drawings, and more particularly to FIG. 1 through FIG. 10, where similar reference characters denote corresponding features consistently throughout the figures, there are shown preferred embodiments and these embodiments are described in the context of the following exemplary system and/or method.

FIG. 1 is an exemplary block diagram of a system 100 for identifying candidate genome sequences by estimating coverage in accordance with some embodiments of the present disclosure.

In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of the system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The I/O interface(s) 106 can include a variety of software and hardware interfaces, for example, a web interface, a graphical user interface, a touch user interface (TUI) and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices (nodes) of the system 100 to one another or to another server.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 108 configured to include information regarding for identifying candidate genome sequences by estimating coverage. The memory 102 may comprise information pertaining to input(s)/output(s) of each step performed by the processor(s) 104 of the system 100 and methods of the present disclosure. In an embodiment, the database 108 may be external (not shown) to the system 100 and coupled to the system via the I/O interface 106.

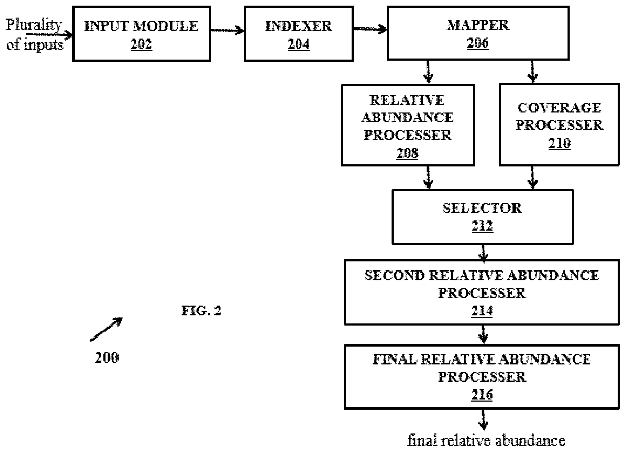

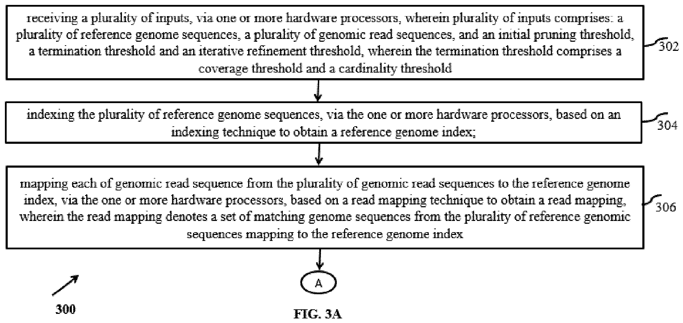

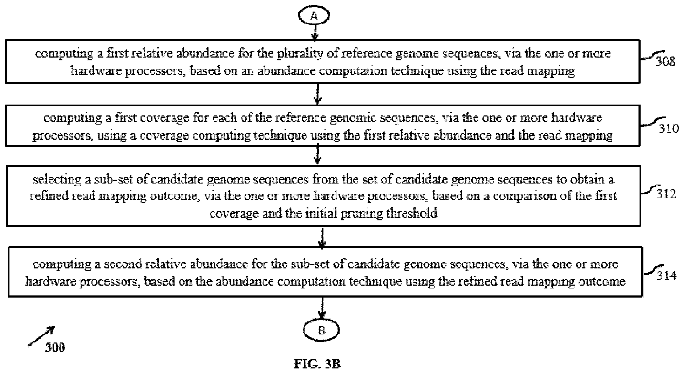

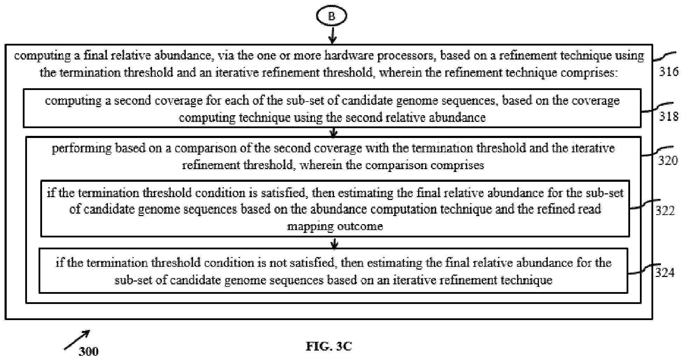

Functions of the components of system 100 are explained in conjunction with functional overview of the system 100 in FIG.2 and flow diagram of FIG.3A, FIG.3B and FIG.3C for identifying candidate genome sequences by estimating coverage.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

FIG.2 is an example functional block diagram of the various modules of the system of FIG. 1, in accordance with some embodiments of the present disclosure. As depicted in the architecture, the FIG.2 illustrates the modules of the system 100 that includes for identifying candidate genome sequences by estimating coverage.

As depicted in FIG.2, the functional system 200 of system 100 system 200 is configured for identifying candidate genome sequences by estimating coverage.

The system 200 comprises an input module 202 configured for receiving a plurality of inputs, wherein plurality of inputs comprises a plurality of reference genome sequences, a plurality of genomic read sequences, and an initial pruning threshold, a termination threshold and an iterative refinement threshold, wherein the termination threshold comprises a coverage threshold and a cardinality threshold. The system 200 further comprises an indexer 204 configured for indexing the plurality of reference genome sequences, based on an indexing technique to obtain a reference genome index. The system 200 further comprises a mapper 206 configured for mapping each of genomic read sequence from the plurality of genomic read sequences to the reference genome index based on a read mapping technique to obtain a read mapping, wherein the read mapping denotes a set of matching genome sequences from the plurality of reference genomic sequences mapping to the reference genome index. The system 200 further comprises a relative abundance processer 208 configured for computing a first relative abundance for the plurality of reference genome sequences, based on an abundance computation technique using the read mapping. The system 200 further comprises a coverage processer 210 configured for computing a first coverage for each of the reference genomic sequences, using a coverage computing technique using the first relative abundance and the read mapping. The system 200 further comprises a selector 212 configured for selecting a sub-set of candidate genome sequences from the set of candidate genome sequences to obtain a refined read mapping outcome, based on a comparison of the first coverage and the initial pruning threshold. The system 200 further comprises a second relative abundance processer 214 configured for computing a second relative abundance for the sub-set of candidate genome sequences, based on the abundance computation technique using the refined read mapping outcome. The system 200 further comprises a final relative abundance processesser 216 configured for computing a final relative abundance based on a refinement technique using the termination threshold and an iterative refinement threshold.

The various modules of the system 100 and the functional blocks in FIG.2 are configured for identifying candidate genome sequences by estimating coverage are implemented as at least one of a logically self-contained part of a software program, a self-contained hardware component, and/or, a self-contained hardware component with a logically self-contained part of a software program embedded into each of the hardware component that when executed perform the above method described herein.

Functions of the components of the system 200 are explained in conjunction with functional modules of the system 100 stored in the memory 102 (not shown) and further explained in conjunction with flow diagram of FIGS.3A-3C. The FIGS.3A-3C with reference to FIG.1, is an exemplary flow diagram illustrating a method 300 for identifying candidate genome sequences by estimating coverage using the system 100 of FIG.1 according to an embodiment of the present disclosure.

The steps of the method of the present disclosure will now be explained with reference to the components of the system 100 of FIG. 1 for identifying candidate genome sequences by estimating coverage and the modules 202-216 as depicted in FIG.2 and the flow diagrams as depicted in FIGS.3A-3C. Although process steps, method steps, techniques or the like may be described in a sequential order, such processes, methods and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 302 of the method 300, a plurality of inputs is received at the input module 202. The plurality of inputs comprises:

> a) a plurality of reference genome sequences,
> b) a plurality of genomic read sequences, and
> c) an initial pruning threshold, a termination threshold and an iterative refinement threshold, wherein the termination threshold comprises a coverage threshold and a cardinality threshold.

In an embodiment, the plurality of reference genome sequences is obtained from plurality of publicly available sources for biomedical data, which include National Center for Biotechnology Information (NCBI), Ensemble, UCSC Genome browser etc. In an example scenario, the plurality of reference microbial sequences is collected from Refseq, Mosaic Challenge and consisted of 24,146 strains from 13,168 species, wherein the species were from 3,559 genus belonging to Virus, Bacteria, Archae, Fungi and Protozoa with an index size of â^¼38 GB. In an example scenario - The plurality of reference microbial sequences is string of characters comprising of a string of characters including "ATGCN".

In an example scenario, the plurality of reference genome sequences is represented as S;

$$S = \{S_1, \ldots S_N\} \qquad (1)$$

Where, N is a collection of reference genome sequences.

For each reference genome sequence (S), there is an associated internal unique reference identifier and S is sequences are a string of characters. The mapping between the internal identifiers and their corresponding external identifier (strain identifier and species identifier) is maintained separately.

Each plurality of reference genome sequence from the plurality of reference genome sequences is associated with:

> (a) a sequence length,
> (b) an internal unique reference identifier, and
> (c) a corresponding external identifier for the internal unique
> reference identifier.

The external identifier comprises a strain identifier and a species identifier, wherein the entire microbial hierarchy or the taxonomic hierarchy can be retrieved using the external taxonomic identifier.

The genomic read sequences is a sample and each genomic read sequence from the plurality of genomic read sequences is associated with a read length and a sequencing depth. In an example scenario - a genomic read sequencing sample is a metagenomic read sample wherein the plurality of microbial read sequences is received/collected from a variety of environment locations such as human body (gut, vaginal, stool etc.), ocean, soil, and many more. The environment samples undergo high throughput sequencing to generate metagenomic read samples using high throughput sequencing platforms including Illumina, Pacbio, Nanopore etc.

Initial pruning threshold is aimed at removing of large fraction of false positives, wherein the initial pruning threshold eliminates the reference genome sequences with extremely low coverage. Further the coverage threshold and the cardinality threshold is an auxiliary information that is known to the user. The cardinality threshold and the coverage threshold allow the user to input the known auxiliary information for accurate estimation and quantification of constituent sequences.

At step 304 of the method 300, the plurality of reference genome sequences is indexed at the indexer 204. The indexing the plurality of reference genome sequences is performed based on an indexing technique to obtain a reference genome index.

In an embodiment, the indexing technique comprises a k-mer indexing technique, one of a FM-indexing technique, a R-indexing technique, a bowtie indexing technique, a suffix tree or suffix array technique, a Rabin-Karp technique, a KMP (Knuth Morris Pratt) technique, a Boyer Moore technique and a De Bruijn Graph.

The reference genome index is a lookup table that associated the plurality of reference genome sequences or subsequence to the reference genome sequence that contains the sequence/subsequence.

Indexing is a technique to efficiently retrieve records form a database based on the attributes associated with indexing requirements. A sequencing alignment technique including a FM-indexing technique, a R-indexing technique, a bowtie indexing technique, a suffix tree or suffix array technique, a Rabin-Karp technique, a KMP (Knuth Morris Pratt) technique, a Boyer Moore technique and a De Bruijn Graph, consists of an indexing and a search function.

In an example scenario, the FM-Indexing technique is used to obtain a reference genome index. The FM indexing technique is a compressed space economical substring index structure based on Burrows Wheeler Transform (BWT). The FM-indexing technique is used to search any exact matching sub-strings in the given text/string and allows compression of the input without compromising the speed of querying.

At step 306 of the method 300, each of genomic read sequence from the plurality of genomic read sequences is mapped to the reference genome index in the mapper 206.The read mapping denotes a set of matching genome sequences from the plurality of reference genomic sequences mapping to the reference genome index. The mapping is performed to obtain a read mapping based on a read mapping technique.

In an embodiment, reads from the plurality of genomic read sequence are sent as a query to the reference genome index. The output of the read mapping Z(R) denotes a set of reference microbial sequence from S to which R maps. The output of the read mapping Z(R) comprises two steps:

1. A portion $C_1 \cup C_2 \ldots C_R$... of the readset (R), and
2. $Q_1 \ldots Q_R$ wherein $Q_i$ is the subset of strains to which every read in the readset $C_i$ maps to.

In an example scenario, the read mapping technique uses a search function of the indexing technique to obtain a read mapping. The read mapping technique perform nearest neighbor search among the reference sequences to identify the candidate genome sequences to which the read has good local alignment. The read mapping technique uses exact search of constituent k-mers in the read to identify the possible target reference sequences.

At step 308 of the method 300, computing a first relative abundance for the plurality of reference genome sequences in the relative abundance processer 208. The first relative abundance is computed based on an abundance computation technique using the read mapping.

In an embodiment, the abundance computation technique comprises one of a Maximum Likelihood Estimation (MLE), a Lasso regression technique, a Bayesian Estimation technique.

In an example scenario, after performing read mapping, the strain level relative abundances are estimated using MLE (Maximum Likelihood Estimation). If $R$ denotes the set of mapped reads and $S$ denote the reference strain collection, the relative abundances of strains is computed in $S$ using the mapping information. Each read r in R is assumed to be sampled independently and randomly from its source strain. The source strain is chosen with probability $a_j$. If $l'_j$ denotes the effective length of strain $s_j$, under the uniform coverage assumption, $l'_j$ is approximated as $l_j - \hat{r}$ where $\hat{r}$ is the average read length. For a given sample, the relative abundances of all the strains is denoted by the vector a where the $i^{th}$ component denotes the relative abundance of the ith reference strain. Clearly, $\Sigma\, a_i = 1$. For a read r, Q(r) denotes the set of strains from S to which r maps. The likelihood for R is computed as shown below:

$$\Pr(\mathcal{R}|a) = \prod_{r \in \mathcal{R}} \Pr(r|a) = \prod_{r \in \mathcal{R}} \sum_{s_j \in Q(r)} (x_j/l'_j) \qquad (2)$$

Wherein,

$$x_j = (a_j l'_j)/\sum_{i=1}^{m} (a_j l'_j)$$

The $x_j$ is simply the length adjusted relative abundance of strain $s_j$ and $\Sigma x_i = 1$. For several reads, corresponding sequence sets $Q(r)$ will be identical wherein, the mapped sequence sets of the reads define a partition of $R = C_i \cup \cdots \cup C_R$ where $C_i$ denotes the subset of reads that are mapped to the same set of sequences denoted as $Q_i$ . Therefore, the

log likelihood with respect to the strain collection S is given by:

$$\mathcal{L}(S) = \sum_{i=1}^{R} n_i \log \left( \sum_{s_j \in Q_i} (x_j / l'_j) \right) \qquad (3)$$

where $n_i$ is the cardinality of $C_i$ and $x_j$ is solved to maximize $L$.

At step 310 of the method 300, a first coverage is computed for each of the reference genomic sequences at the coverage processer 210. The first coverage is computed using a coverage computing technique using the first relative abundance and the read mapping.

In an embodiment, the coverage computing technique is based on the average number of genome read sequences that are mapped to reference genome sequences. The coverage is computed in several steps based on the coverage computing technique as described below:

EM based solution of the original MLE formulation also yields an estimate of the latent matrix $M_{R \times N}$, where $M(i,j)$ is the number of reads from the read partition $C_i$ that have originated from reference genomic sequences $s_j$. All the reads in $C_i$ map to the same subset of strains $Q_i$. If strain $s_j$ does not belong to $Q_i$ then clearly $M(i,j) = 0$. From an optimal MLE solution, estimate for $M(i,j)$, where strain $s_j$ belongs to $Q_i$, is obtained as shown below.

$$M(i,j) = n_i \cdot (x_j / l'_j) / \sum_{s_k \in Q_i} (x_k / l'_k) \qquad (4)$$

Using matrix M, estimate for the total number of reads originating from the reference genomic sequence $s_j$ denoted by $Y_j$ is given by:

$$Y_j = \sum_{i=1}^{R} M(i,j) \qquad (5)$$

also, $Y_j$ and $x_j$ are related as;

$$x_j = Y_j / N_r$$

where $N_r = \sum n_i = |\mathcal{R}|$

Using the estimate of $Y_j$, the coverage of the reference genomic sequence $s_j$, denoted by $cov(j)$, is computed by:

$$cov(j) = Y_j \hat{r} / l'_j \qquad (6)$$

From the above set of equations, the optimal estimates for $x_j$ and $cov(j)$ are related as show below:

$$cov(j) = x_j N_r \hat{r} / l'_j \qquad (7)$$

At step 312 of the method 300, a sub-set of candidate genome sequences is selected from the set of candidate genome sequences in the selector 212. The sub-set of candidate genome sequences is selected, and the selected sub-set of candidate genome sequences is used to obtain a refined read mapping outcome.

The sub-set of candidate genome sequences is selected based on a comparison of the first coverage and the initial pruning threshold.

In an embodiment, the initial pruning threshold is based on a minimum coverage threshold, which eliminates the genome sequences that have low coverage.

A sub-set of candidate genome sequences S' is selected if the coverage of sequence $s_j$ is greater than the initial pruning threshold, wherein for every sequence $j$ present S', if $cov(j) > "t"$ (initial pruning threshold).

The coverage threshold is an auxiliary information for accurate estimation and quantification of constituent sequences.

At step 314 of the method 300, a second relative abundance is computed for the sub-set of candidate genome sequences in the second relative abundance processer 214. The second relative abundance is computed based on the abundance computation technique using the refined read mapping outcome, wherein for the refined read mapping outcome, all the excluded or eliminated reference sequences are removed from the mapped set of references for each read.

In an embodiment, a read r, Q(r) denotes the set of strains from S to which r maps. The likelihood for R is computed as shown below:

$$\Pr(\mathcal{R}|a) = \prod_{r \in \mathcal{R}} \Pr(r|a) = \prod_{r \in \mathcal{R}} \sum_{s_j \in Q(r)} (x_j / l'_j) \qquad (8)$$

Coverage values *cov(j)* for different strains can vary as it depends on both read coverage as well as the abundance of the strain in the sample. In particular, *cov(j)* divided by the read coverage for R is an estimate of the number of copies of the strain. Using equation (6) and noting that $N_r$ and $\hat{r}$ are constants, it is straightforward to verify that at an optimal MLE solution, the objective value $L^o$ is given by:

$$\mathcal{L}^o(S) = \sum_{i=1}^{\mathcal{R}} n_i \log\left(\sum_{s_j \in Q_i} cov(j)\right) - c \qquad (9)$$

When the strain set *S* is restricted to *S'* (sub-set of candidate genome sequences), some of the reads in *R* can become unmapped because none of the mapped strains for this read are present in *S'*. This is a low probability event if *S'* contains all the strains present in the sample. Let $p_\varepsilon$ denote the probability of observing such an unmapped read.

From the independence assumption, the probability of observing t such reads is $p_\varepsilon^t$. Therefore, in the likelihood expression given by equation 2, each unmapped read contributes a multiplicative term $p_\varepsilon$. Consequently, the optimal objective valueLcan be modified as shown below:

$$\mathcal{L}^*(S') = \sum_{Q_i \neq \emptyset} n_i \log\left(\sum_{s_j \in Q_i} cov(j)\right) + \lambda N_\varepsilon - c \qquad (10)$$

At step 316 of the method 300, a final relative abundance is computed in the final relative abundance processer 216. The final relative abundance is computed based on a refinement technique using the termination threshold and an iterative refinement threshold. The refinement technique comprises several steps as described below between steps 318-324.

At step 318 of the method 300, a second coverage is computed for each of the sub-set of candidate genome sequences, based on the coverage computing technique using the second relative abundance.

In an embodiment, the second coverage is computed based on the coverage computing technique as detailed out using equation 4, 5, 6 and 7.

At step 320 of the method 300, the second coverage is compared with the termination threshold and the iterative refinement threshold. Based on the comparison, performing one of step 322 or 324:

At step 322 of the method 300, the final relative abundance is estimated for the sub-set of candidate genome sequences if the termination threshold condition is satisfied. The final relative abundance for the sub-set of candidate genome sequences is estimated based on the abundance computation technique and the refined read mapping outcome.

In an embodiment, a termination threshold condition consists of a cardinality threshold and a coverage threshold. A user can select either or both the thresholds as a termination threshold condition. For every reference genome sequence j in S', the termination threshold condition is satisfied (a) if *Cov(j) > coverage threshold* and/or (b) *Cardinality of S' > cardinality threshold,* then the final relative abundance is computed for every j in S.

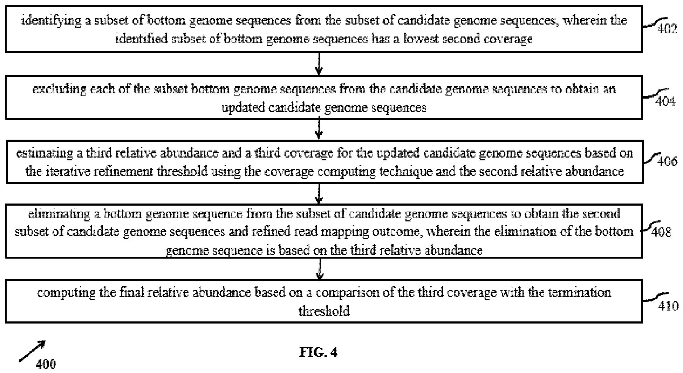

At step 324 of the method 300, the final relative abundance is estimated for the sub-set of candidate genome sequences if the termination threshold condition is not satisfied. The final relative abundance is estimated based on an iterative refinement technique. The iterative refinement technique includes several steps as illustrated in the flowchart (400) of FIG.4.

At step 402 of the method 400, a subset of bottom genome sequences is identified from the subset of candidate genome sequences. The identified subset of bottom genome sequences has a lowest second coverage.

In an embodiment, a bottom subset of sequences comprises of "b" number of sequences which have the lowest second coverage is identified from *S'* the bottom subset of sequences $s'_j$ for $j = \{1... . b\}$.

At step 404 of the method 400, each of the bottom subset genome sequences is excluded from the candidate genome sequences to obtain an updated candidate genome sequence.

In an embodiment, for an example scenario wherein for each of the bottom b candidates

$$s'_j \; for \; j = \{1 \dots b\}$$ , a subset of candidate genome sequences is computed as ($L^*(S'\backslash s_j)$).

Among these b candidates - the bottom genome sequences $s'_j$ corresponding to the largest ($L^*(S'\backslash s_j)$) value are excluded to obtain the updated candidate genome set.

At step 406 of the method 400, a third relative abundance and a third coverage are estimated for the updated candidate genome sequences. The third relative abundance and a third coverage is estimated based on the iterative refinement threshold using the coverage computing technique and the second relative abundance.

As a first step, if a number of iterations (represented as an iteration step) is greater than the iterative refinement threshold then the second relative abundance is updated using abundance computing technique and the refined read mapping outcome, after which the iteration step is reset to zero. Further, the third abundance is estimated using the coverage computing technique and the second relative abundance. In an embodiment, if the iteration step is greater than the iterative refinement threshold then the third abundance is estimated using the coverage computing technique.

In an embodiment, each of the bottom b candidates $s'_j \; for \; j =$ {1 ... . b}, ($L^*(S'\backslash s_j)$) value is estimated using the coverage computing technique using the equations 4,5, 6 and 7.

At step 408 of the method 400, a bottom genome sequence is eliminated from the subset of candidate genome sequences. The elimination of the bottom genome sequence is based on the third relative abundance. The bottom genome sequence is eliminated to obtain the second subset of candidate genome sequences and refined read mapping outcome.

In an embodiment, for an example scenario, among these *b* candidates $s'_j$ corresponding to the largest ($L^*(S'\backslash s_j)$) value is eliminated to obtain the updated candidate genome set.

At step 410 of the method 400, the final relative abundance is computed based on a comparison of the third coverage with the termination threshold.

In an embodiment, final relative abundance is computed based on a comparison of the third coverage with the termination threshold using the termination threshold condition that consists of a cardinality threshold and a coverage threshold. A user can select either or both the thresholds as the termination threshold condition. Based on the termination threshold condition, the final relative abundance is computed for every j in reference genome sequence j in S' if (a) *Cov(j) > coveragethreshold* and/or (b) *CardinalityofS > cardinalitythreshold*.

EXPERIMENTS:

A wide variety of metrics are utilized to compare the profiling outputs with the ground truth. The relative abundance distributions are compared using well-known divergence measures for probability distributions to compare abundance distributions, the Jensen-Shannon divergence, Total variation distance, Hellinger distance and cumulative abundance mass. The results are simulated for the above test data using the disclosed technique and the performance is documented at strain level and species level.

To compare the profiling outputs on the ability to correctly identify the strains and species, recall and F1-score metrics is compared using the well-known precision techniques. The results are simulated for the above test data using the disclosed technique and the performance is documented at strain level and species level.

For three synthetic datasets namely, High complexity (HC)-1, HC-2, and Low complexity (LC) is simulated to generate Illumina paired end reads of 150bp length and with 20X coverage. Both HC-1 and HC-2 datasets are high complexity metagenomic samples, where multiple strains from same species or species with same genus are present in a sample, such that the constituent strains bear high similarity. The LC dataset is a low complexity data with consisting of 50 strains from 41 species in total and spanning 38 genera.HC-1 and HC-2 are high complexity datasets, where HC-1 consists of 34 strains from 10 species spanning 8 genera and HC-2 consists of 60 strains from 25 species spanning 25 genera. The strain level output of MetaPhlAn2 was mapped to the NCBI taxonomy to compare them with the ground truth. The results are tabulated in below sections.

Table 1 compares the strain level profiling performance of our invention with the current state of art tool which is MetaPhlAn2 on LC dataset. The strain level profiling performance is compared based on variety of measures. JSD, TVD and Hellinger Distance denote better performance if the value is lower, while for Total Mass, Precision, Recall and F 1-score higher values denote better performance.

Table 1 - Strain level profiling performances on LC dataset

| LC | | |
|---|---|---|
| Tool | Invention | MetaPhlAn2 |
| JSD | 0.1587 | 0.7877 |
| TVD | 0.0692 | 0.9154 |
| Hellinger_Distance | 0.1830 | 0.9453 |
| Total Mass | 0.9470 | 0.1353 |
| Prec | 0.9091 | 0.2000 |
| Recall | 1.0000 | 0.1000 |
| F1 | 0.9524 | 0.1333 |

Table 2 compares the strain level profiling performance of our invention with the current state of art tool which is MetaPhlAn2 on HC-1 dataset. The strain level profiling performance is compared based on variety of measures. JSD, TVD and Hellinger Distance denote better performance if the value is lower, while for Total Mass, Precision, Recall and F 1-score higher values denote better performance.

Table 2: Strain level profiling performances on HC-1 dataset

| Tool | Invention | MetaPhlAn2 |
|---|---|---|
| JSD | 0.198200329095517 | 0.819663606012411 |
| TVD | 0.074646599999999 | 0.971428571428578 |
| Hellinger_Distance | 0.237749234930645 | 0.984497423962273 |
| Total Mass | 0.9447686 | 0.0331266 |
| Prec | 0.941176470588235 | 0.111111111111111 |
| Recall | 0.941176470588235 | 0.029411764705882 |
| F1 | 0.941176470588235 | 0.046511627906977 |

Table.3 compares the strain level profiling performance of our invention with the current state of art tool which is MetaPhlAn2 on HC-2 dataset. The strain level profiling performance is compared on variety of measures. JSD, TVD and Hellinger Distance denote better performance if the value is lower, while for Total Mass, Precision, Recall and F 1-score higher values denote better performance.

Table 3: Strain level profiling performances on HC-2 dataset.

| Tool | Invention | MetaPhlAn2 |
|---|---|---|
| JSD | 0.299154471514847 | 0.813892551567749 |
| TVD | 0.15568543 | 0.95880335 |
| Hellinger_Distance | 0.357664914724235 | 0.977552802221621 |
| Total Mass | 0.88604082 | 0.0436079 |
| Prec | 0.819672131147541 | 0.071428571428572 |
| Recall | 0.833333333333333 | 0.033333333333333 |
| F1 | 0.826446280991735 | 0.045454545454546 |

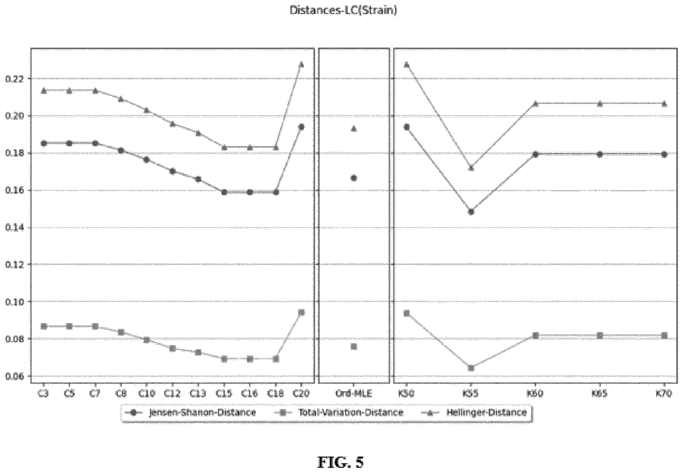

The FIG.5 compares the performance of the disclosed techniques with the regular MLE in quantifying the abundance at the level of strains on LC dataset. The performance with three distance measures is compared and described. The Coverage (C) and the cardinality(C) are user input auxiliary information. The disclosed techniques have been tested with various the coverage (C) and the cardinality parameters (K), smaller value of the distance measures signifies better performance.

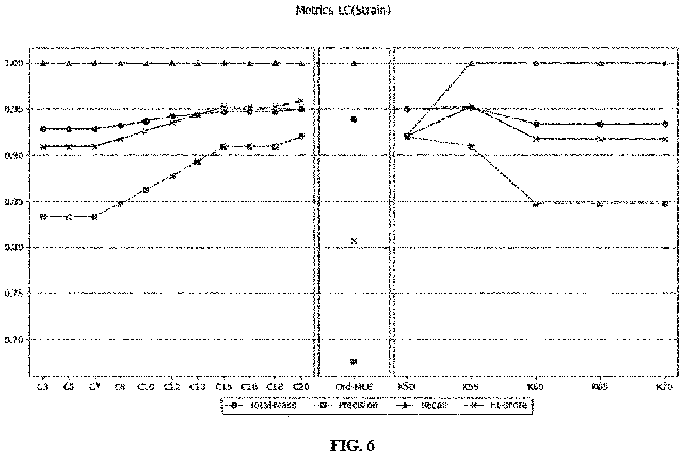

The FIG.6 compares the performance of the disclosed techniques with the regular MLE in profiling the metagenomic samples at the level of strains on LC dataset. The performance with well-known precision, recall and F 1 score is compared. The total mass metrics for comparison. The coverage (C) and the cardinality(C) are user input auxiliary information. The disclosed techniques have been tested with various coverage (C) and cardinality parameters(K) has been tested, larger value of the metrics denotes better performance.

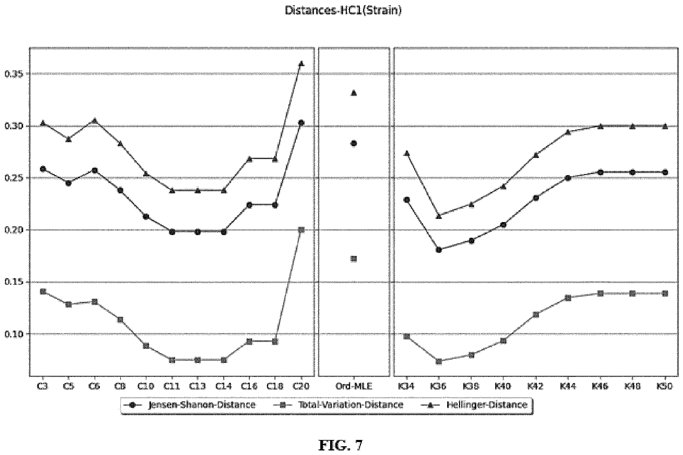

The FIG.7 compares the performance of the disclosed techniques with the regular MLE in quantifying the abundance at the level of strains on HC-1 dataset. The performance with three distance measures is compared and described. The coverage (C) and the cardinality(C) are user input auxiliary information. The disclosed techniques have been tested with various the coverage (C) and the cardinality parameters (K) has been tested; smaller value of the distance measures signifies better performance.

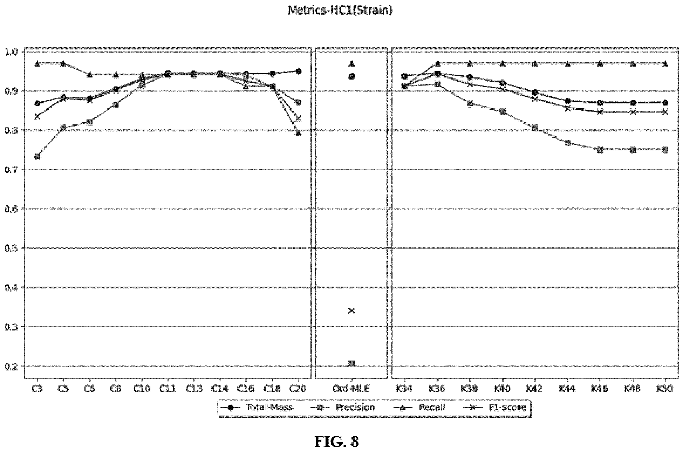

The FIG.8 compares the performance of the disclosed techniques with the regular MLE in profiling the metagenomic samples at the level of strains on HC-1 dataset. The performance is compared with well-known precision, recall and F1 score. Further total mass metrics is also utilized for comparison. The coverage (C) and the cardinality(C) are user input auxiliary information. The disclosed techniques have been tested with various the coverage (C) and the cardinality parameters (K), larger value of the metrics denotes better performance.

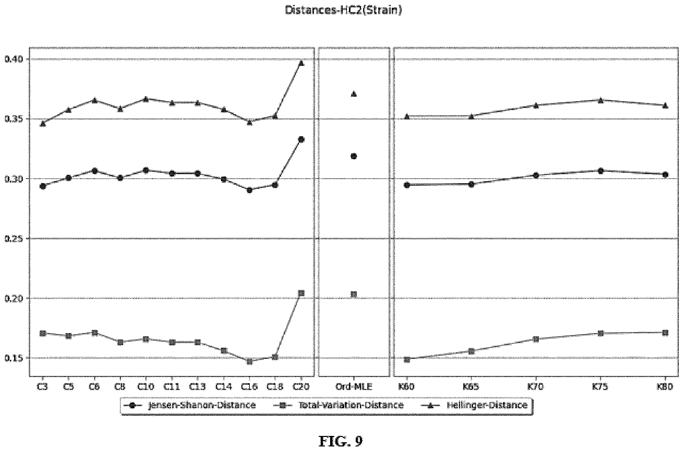

The FIG.9 compares the performance of the disclosed techniques with the regular MLE in quantifying the abundance at the level of strains on HC-2 dataset. We compare the performance with three distance measures described above. The coverage (C) and the cardinality(C) are user input auxiliary information. The disclosed techniques have been tested with various the coverage (C) and the cardinality parameters (K), smaller value of the distance measures signifies better performance.

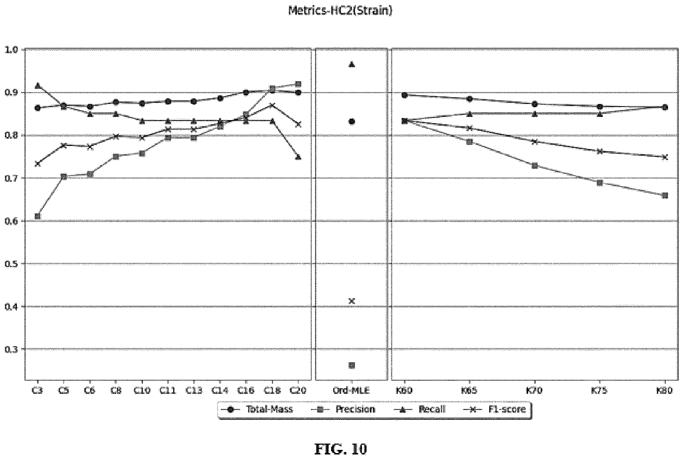

The FIG. 10 compares the performance of the disclosed techniques with the regular MLE in profiling the metagenomic samples at the level of strains on HC-2 dataset. We compare the performance with well-known precision, recall and F1 score. The total mass metrics for comparison is also used. The coverage (C) and the cardinality(C) are user input auxiliary information. The disclosed techniques have been tested with various the coverage (C) and the cardinality parameters (K); larger value of the metrics denotes better performance.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

This disclosure relates generally relates to identifying candidate genome sequences. Next generation sequencing (NGS) is a massively parallel sequencing technique for identifying candidate genome sequences. The current state-of-the-art techniques for identifying candidate genome sequences does not efficiently address the problem of distributing abundance values across several related strains that are present in the reference under the same species. The disclosed technique proposes a technique for identifying candidate genome sequences by estimating coverage. The disclosed technique includes a local search-based optimization to compute maximum likelihood-based estimates using constrains on coverage/cardinality thresholds for identifying candidate genome sequences.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.  A processor implemented method (300), comprising:

    receiving a plurality of inputs (302), via one or more hardware processors, wherein plurality of inputs comprises:

    a plurality of reference genome sequences,
    a plurality of genomic read sequences, and
    an initial pruning threshold, a termination threshold and an iterative refinement threshold, wherein the termination threshold comprises a coverage threshold and a cardinality threshold;

    indexing the plurality of reference genome sequences, via the one or more hardware processors, based on an indexing technique to obtain a reference genome index (304);
    mapping each of genomic read sequence from the plurality of genomic read sequences to the reference genome index, via the one or more hardware processors, based on a read mapping technique to obtain a read mapping, wherein the read mapping denotes a set of matching genome sequences from the plurality of reference genomic sequences mapping to the reference genome index (306);
    computing a first relative abundance for the plurality of reference genome sequences, via the one or more hardware processors, based on an abundance computation technique using the read mapping (308);
    computing a first coverage for each of the reference genomic sequences, via the one or more hardware processors, using a coverage computing technique using the first relative abundance and the read mapping (310);
    selecting a sub-set of candidate genome sequences from the set of candidate genome sequences to obtain a refined read mapping outcome, via the one or more hardware processors, based on a comparison of the first coverage and the initial pruning threshold (312);
    computing a second relative abundance for the sub-set of candidate genome sequences, via the one or more hardware processors, based on the abundance computation technique using the refined read mapping outcome (314); and
    computing a final relative abundance (316), via the one or more hardware processors, based on a refinement technique using the termination threshold and an iterative refinement threshold, wherein the refinement technique comprises:

    computing a second coverage for each of the sub-set of candidate genome sequences, based on the coverage computing technique using the second relative abundance (318); and
    performing (320) based on a comparison of the second coverage with the termination threshold and the iterative refinement threshold one of:

    if the termination threshold condition is satisfied, then estimating the final relative abundance for the sub-set of candidate genome sequences based on the abundance computation technique and the refined read mapping outcome (322); and

if the termination threshold condition is not satisfied, then estimating the final relative abundance for the sub-set of candidate genome sequences based on an iterative refinement technique (324).

2. The processor implemented method of claim 1, wherein the indexing technique comprises a k-mer indexing technique, one of a FM-indexing technique, a R-indexing technique, a bowtie indexing technique, a suffix tree or suffix array technique, a Rabin-Karp technique, a KMP (Knuth Morris Pratt) technique, a Boyer Moore technique and a De Bruijn Graph.

3. The processor implemented method of claim 1, wherein the abundance computation technique comprises one of a Maximum Likelihood Estimation (MLE), a Lasso regression technique, a Bayesian Estimation technique.

4. The processor implemented method of claim 1, wherein the coverage computing technique is the average number of genome read sequences that are mapped to reference genome sequences.

5. The processor implemented method of claim 1, wherein the iterative refinement technique (400) is iteratively performed for computing the final relative abundance based on the termination threshold and the iterative refinement threshold, wherein the iterative refinement technique comprises:

identifying a subset of bottom genome sequences from the subset of candidate genome sequences, wherein the identified subset of bottom genome sequences has a lowest second coverage (402);
excluding each of the subset bottom genome sequences from the candidate genome sequences to obtain an updated candidate genome sequences (404);
estimating a third relative abundance and a third coverage for the updated candidate genome sequences based on the iterative refinement threshold using the coverage computing technique and the second relative abundance (406);
eliminating a bottom genome sequence from the subset of candidate genome sequences to obtain the second subset of candidate genome sequences and refined read mapping outcome, wherein the elimination of the bottom genome sequence is based on the third relative abundance (408); and
computing the final relative abundance based on a comparison of the third coverage with the termination threshold (410).

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive a plurality of inputs, via one or more hardware processors, wherein plurality of inputs comprises:

a plurality of reference genome sequences,
a plurality of genomic read sequences, and
an initial pruning threshold, a termination threshold and an iterative refinement threshold, wherein the termination threshold comprises a coverage threshold and a cardinality threshold;

index the plurality of reference genome sequences, via the one or more hardware processors, based on an indexing technique to obtain a reference genome index;
map each of genomic read sequence from the plurality of genomic read sequences to the reference genome index, via the one or more hardware processors, based on a read mapping technique to obtain a read mapping, wherein the read mapping denotes a set of matching genome sequences from the plurality of reference genomic sequences mapping to the reference genome index;
computing a first relative abundance for the plurality of reference genome sequences, via the one or more hardware processors, based on an abundance computation technique using the read mapping;
compute a first coverage for each of the reference genomic sequences, via the one or more hardware processors, using a coverage computing technique using the first relative abundance and the read mapping;
select a sub-set of candidate genome sequences from the set of candidate genome sequences to obtain a refined read mapping outcome, via the one or more hardware processors, based on a comparison of the first coverage and the initial pruning threshold;

compute a second relative abundance for the sub-set of candidate genome sequences, via the one or more hardware processors, based on the abundance computation technique using the refined read mapping outcome; and

compute a final relative abundance, via the one or more hardware processors, based on a refinement technique using the termination threshold and an iterative refinement threshold, wherein the refinement technique comprises:

compute a second coverage for each of the sub-set of candidate genome sequences, based on the coverage computing technique using the second relative abundance; and

perform based on a comparison of the second coverage with the termination threshold and the iterative refinement threshold, one of:

if the termination threshold condition is satisfied, then estimating the final relative abundance for the sub-set of candidate genome sequences based on the abundance computation technique and the refined read mapping outcome; and

if the termination threshold condition is not satisfied, then estimating the final relative abundance for the sub-set of candidate genome sequences based on an iterative refinement technique.

7. The system of claim 6, wherein the indexing technique comprises a k-mer indexing technique, one of a FM-indexing technique, a R-indexing technique, a bowtie indexing technique, a suffix tree or suffix array technique, a Rabin-Karp technique, a KMP (Knuth Morris Pratt) technique, a Boyer Moore technique and a De Bruij n Graph.

8. The system of claim 6, wherein the abundance computation technique comprises one of a Maximum Likelihood Estimation (MLE), a Lasso regression technique, a Bayesian Estimation technique.

9. The system of claim 6, wherein the coverage computing technique is the average number of genome read sequences that are mapped to reference genome sequences.

10. The system of claim 6, wherein the iterative refinement technique is iteratively performed for computing the final relative abundance based on the termination threshold and the iterative refinement threshold, wherein the iterative refinement technique comprises:

identifying a subset of bottom genome sequences from the subset of candidate genome sequences, wherein the identified subset of bottom genome sequences has a lowest second coverage;

excluding each of the subset bottom genome sequences from the candidate genome sequences to obtain an updated candidate genome sequences;

estimating a third relative abundance and a third coverage for the updated candidate genome sequences based on the iterative refinement threshold using the coverage computing technique and the second relative abundance;

eliminating a bottom genome sequence from the subset of candidate genome sequences to obtain the second subset of candidate genome sequences and refined read mapping outcome, wherein the elimination of the bottom genome sequence is based on the third relative abundance; and

computing the final relative abundance based on a comparison of the third coverage with the termination threshold.

11. One or more non-transitory machine-readable information storage mediums comprising one or more instructions which when executed by one or more hardware processors cause:

receiving a plurality of inputs, wherein plurality of inputs comprises:

a plurality of reference genome sequences,
a plurality of genomic read sequences, and
an initial pruning threshold, a termination threshold and an iterative refinement threshold, wherein the termination threshold comprises a coverage threshold and a cardinality threshold;

indexing the plurality of reference genome sequences based on an indexing technique to obtain a reference genome index;

mapping each of genomic read sequence from the plurality of genomic read sequences to the reference genome index based on a read mapping technique to obtain a read mapping, wherein the read mapping denotes a set of matching genome sequences from the plurality of reference genomic sequences mapping to the reference genome index;

computing a first relative abundance for the plurality of reference genome sequences based on an abundance computation technique using the read mapping;

computing a first coverage for each of the reference genomic sequences using a coverage computing technique using the first relative abundance and the read mapping;

selecting a sub-set of candidate genome sequences from the set of candidate genome sequences to obtain a refined read mapping outcome based on a comparison of the first coverage and the initial pruning threshold;

computing a second relative abundance for the sub-set of candidate genome sequences based on the abundance computation technique using the refined read mapping outcome; and

computing a final relative abundance based on a refinement technique using the termination threshold and an iterative refinement threshold, wherein the refinement technique comprises:

> computing a second coverage for each of the sub-set of candidate genome sequences, based on the coverage computing technique using the second relative abundance; and
>
> performing based on a comparison of the second coverage with the termination threshold and the iterative refinement threshold one of:
>
>> if the termination threshold condition is satisfied, then estimating the final relative abundance for the sub-set of candidate genome sequences based on the abundance computation technique and the refined read mapping outcome; and
>>
>> if the termination threshold condition is not satisfied, then estimating the final relative abundance for the sub-set of candidate genome sequences based on an iterative refinement technique.

12. The one or more non-transitory machine readable information storage mediums of claim 11, wherein the indexing technique comprises a k-mer indexing technique, one of a FM-indexing technique, a R-indexing technique, a bowtie indexing technique, a suffix tree or suffix array technique, a Rabin-Karp technique, a KMP (Knuth Morris Pratt) technique, a Boyer Moore technique and a De Bruijn Graph.

13. The one or more non-transitory machine readable information storage mediums of claim 11, wherein the abundance computation technique comprises one of a Maximum Likelihood Estimation (MLE), a Lasso regression technique, a Bayesian Estimation technique.

14. The one or more non-transitory machine readable information storage mediums of claim 11, wherein the coverage computing technique is the average number of genome read sequences that are mapped to reference genome sequences.

15. The one or more non-transitory machine readable information storage mediums of claim 11, wherein the iterative refinement technique is iteratively performed for computing the final relative abundance based on the termination threshold and the iterative refinement threshold, wherein the iterative refinement technique comprises:

> identifying a subset of bottom genome sequences from the subset of candidate genome sequences, wherein the identified subset of bottom genome sequences has a lowest second coverage;
>
> excluding each of the subset bottom genome sequences from the candidate genome sequences to obtain an updated candidate genome sequences;
>
> estimating a third relative abundance and a third coverage for the updated candidate genome sequences based on the iterative refinement threshold using the coverage computing technique and the second relative abundance;
>
> eliminating a bottom genome sequence from the subset of candidate genome sequences to obtain the second subset of candidate genome sequences and refined read mapping outcome, wherein the elimination of the bottom genome sequence is based on the third relative abundance; and
>
> computing the final relative abundance based on a comparison of the third coverage with the termination threshold.

SYSTEM
100

PROCESSOR(S)
104

I/O
INTERFACE(S)
106

MEMORY
102

DATABASE
108

FIG. 1

Plurality of inputs → INPUT MODULE 202 → INDEXER 204 → MAPPER 206 → RELATIVE ABUNDANCE PROCESSER 208 / COVERAGE PROCESSER 210 → SELECTOR 212 → SECOND RELATIVE ABUNDANCE PROCESSER 214 → FINAL RELATIVE ABUNDANCE PROCESSER 216 → final relative abundance

FIG. 2

200

receiving a plurality of inputs, via one or more hardware processors, wherein plurality of inputs comprises: a plurality of reference genome sequences, a plurality of genomic read sequences, and an initial pruning threshold, a termination threshold and an iterative refinement threshold, wherein the termination threshold comprises a coverage threshold and a cardinality threshold

302

indexing the plurality of reference genome sequences, via the one or more hardware processors, based on an indexing technique to obtain a reference genome index;

304

mapping each of genomic read sequence from the plurality of genomic read sequences to the reference genome index, via the one or more hardware processors, based on a read mapping technique to obtain a read mapping, wherein the read mapping denotes a set of matching genome sequences from the plurality of reference genomic sequences mapping to the reference genome index

306

A

300

FIG. 3A

(A)

computing a first relative abundance for the plurality of reference genome sequences, via the one or more hardware processors, based on an abundance computation technique using the read mapping — 308

computing a first coverage for each of the reference genomic sequences, via the one or more hardware processors, using a coverage computing technique using the first relative abundance and the read mapping — 310

selecting a sub-set of candidate genome sequences from the set of candidate genome sequences to obtain a refined read mapping outcome, via the one or more hardware processors, based on a comparison of the first coverage and the initial pruning threshold — 312

computing a second relative abundance for the sub-set of candidate genome sequences, via the one or more hardware processors, based on the abundance computation technique using the refined read mapping outcome — 314

(B)

300

FIG. 3B

B

computing a final relative abundance, via the one or more hardware processors, based on a refinement technique using the termination threshold and an iterative refinement threshold, wherein the refinement technique comprises: — 316

computing a second coverage for each of the sub-set of candidate genome sequences, based on the coverage computing technique using the second relative abundance — 318

performing based on a comparison of the second coverage with the termination threshold and the iterative refinement threshold, wherein the comparison comprises — 320

if the termination threshold condition is satisfied, then estimating the final relative abundance for the sub-set of candidate genome sequences based on the abundance computation technique and the refined read mapping outcome — 322

if the termination threshold condition is not satisfied, then estimating the final relative abundance for the sub-set of candidate genome sequences based on an iterative refinement technique — 324

300

FIG. 3C

| identifying a subset of bottom genome sequences from the subset of candidate genome sequences, wherein the identified subset of bottom genome sequences has a lowest second coverage | 402 |

| excluding each of the subset bottom genome sequences from the candidate genome sequences to obtain an updated candidate genome sequences | 404 |

| estimating a third relative abundance and a third coverage for the updated candidate genome sequences based on the iterative refinement threshold using the coverage computing technique and the second relative abundance | 406 |

| eliminating a bottom genome sequence from the subset of candidate genome sequences to obtain the second subset of candidate genome sequences and refined read mapping outcome, wherein the elimination of the bottom genome sequence is based on the third relative abundance | 408 |

| computing the final relative abundance based on a comparison of the third coverage with the termination threshold | 410 |

400

FIG. 4

Distances-LC(Strain)

**FIG. 5**

Metrics-LC(Strain)

FIG. 6

Distances-HC1(Strain)

FIG. 7

Metrics-HC1(Strain)

FIG. 8

26

Distances-HC2(Strain)

**FIG. 9**

Metrics-HC2(Strain)

FIG. 10

Europäisches
Patentamt

European
Patent Office

Office européen
des brevets

# EUROPEAN SEARCH REPORT

**Application Number**

EP 23 19 7703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TRUONG DUY TIN ET AL: "MetaPhlAn2 for enhanced metagenomic taxonomic profiling", NATURE METHODS, vol. 12, no. 10, 29 September 2015 (2015-09-29), pages 902-903, XP093134349, New York ISSN: 1548-7091, DOI: 10.1038/nmeth.3589 * whole document * | 1-15 | INV.<br>G16B30/00<br>G16B30/10 |
| A | TRUONG DUY TIN ET AL: "Supplementary Information of MetaPhlAn2 for enhanced metagenomic taxonomic profiling", NATURE METHODS, vol. 12, no. 10, 29 September 2015 (2015-09-29), pages 902-903, XP093134354, New York ISSN: 1548-7091, DOI: 10.1038/nmeth.3589 * the whole document * | 1-15 | |
| | ───── | | TECHNICAL FIELDS SEARCHED (IPC) |
| A | DERRICK E WOOD ET AL: "Kraken: ultrafast metagenomic sequence classification using exact alignments", GENOME BIOLOGY, vol. 15, no. 3, 1 January 2014 (2014-01-01), page R46, XP055282331, ISSN: 1465-6906, DOI: 10.1186/gb-2014-15-3-r46 * whole document, in particular Figures; page 1, column 2, last paragraph- page 2, column 2, first paragraph; Materials and Methods * | 1-15 | G16B |

─────

-/--

The present search report has been drawn up for all claims

| Place of search | Date of completion of the search | Examiner |
|---|---|---|
| Munich | 26 February 2024 | Vanmontfort, D |

EPO FORM 1503 03.82 (P04C01)

Europäisches
Patentamt

European
Patent Office

Office européen
des brevets

# EUROPEAN SEARCH REPORT

Application Number

EP 23 19 7703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WOOD DERRICK E. ET AL: "Improved metagenomic analysis with Kraken 2", GENOME BIOLOGY 2015, vol. 20, no. 1, 28 November 2019 (2019-11-28), XP093134157, London, UK ISSN: 1474-760X, DOI: 10.1186/s13059-019-1891-0 * wholde document, in particular abstract; Figure 1: page 1, column 1, paragraph 1- page 2, column 1, paragraph 2 * | 1-15 | |
| A | MARIC J ET AL: "Approaches to metagenomic classification and assembly", 2019 42ND INTERNATIONAL CONVENTION ON INFORMATION AND COMMUNICATION TECHNOLOGY, ELECTRONICS AND MICROELECTRONICS (MIPRO), CROATIAN SOCIETY MIPRO, 20 May 2019 (2019-05-20), pages 348-356, XP033574703, DOI: 10.23919/MIPRO.2019.8756644 [retrieved on 2019-07-08] * whole document, in particular Figures, paragraphs "II. Methodology" and "III. tools" * | 1-15 | |

-----

-----

-/--

**TECHNICAL FIELDS SEARCHED (IPC)**

The present search report has been drawn up for all claims

| Place of search | Date of completion of the search | Examiner |
|---|---|---|
| Munich | 26 February 2024 | Vanmontfort, D |

EPO FORM 1503 03.82 (P04C01)

Europäisches
Patentamt

European
Patent Office

Office européen
des brevets

# EUROPEAN SEARCH REPORT

Application Number

EP 23 19 7703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WALIA V ET AL: "MAGE: Strain Level Profiling of Metagenome Samples" In: "Comparative Genomics, 20TH INTERNATIONAL CONFERENCE, RECOMB-CG 2023, Istanbul Turkey, April 14-15 2023, Proceedings", 13 July 2023 (2023-07-13), LECTURE NOTES IN COMPUTER SCIENCE (INCLUDING SUBSERIES LECTURE NOTES IN ARTIFICIAL INTELLIGENCE AND LECTURE NOTES IN BIOINFORMATICS) – COMPARATIVE GENOMICS – 20TH INTERNATIONAL CONFERENCE, RECOMB-CG 2023, PROCEEDINGS 2023 SPRINGER SCIENCE AND BUSINES, XP002811102, pages 215-231, * the whole document * | 1-15 | |

TECHNICAL FIELDS
SEARCHED (IPC)

The present search report has been drawn up for all claims

| Place of search | Date of completion of the search | Examiner |
|---|---|---|
| Munich | 26 February 2024 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221058653